# EUROPEAN PATENT SPECIFICATION

(11) **EP 1 381 340 B1**
(45) Date of publication and mention of the grant of the patent: **21.03.2012**
(21) Application number: 02765984.6
(22) Date of filing: 13.08.2002
(51) Int. Cl.: A61F 13/15

(54) **MULTIPLE ZONE APERTURED WEB**
MEHRZONEN - PERFORIERTE SCHICHT
BANDE PERFOREE MULTIZONES

(30) Priority: 14.08.2001 US 312330 P
(43) Date of publication of application: 21.01.2004
(73) Proprietor: McNeil-PPC, Inc., Skillman, NJ 08558 (US); Johnson, Philip S., New Brunswick, NJ 08933 (US)
(72) Inventor: JOHNSON, Philip , S., c/o Johnson & Johnson, New Brunswick, NJ 08933 (US); GUBERNICK, David, Cherry Hill, NJ 08003 (US); KELLY, William, G., F., Middlesex, NJ 08846 (US)
(74) Representative: Mercer, Christopher Paul
(86) International application number: PCT/US2002/025672
(87) International publication number: WO 2003/015681

(56) References cited:
- EP-A- 0 165 807
- EP-A- 0 761 190
- WO-A-97/40793
- GB-A- 2 310 606
- US-A- 4 609 518
- US-A- 5 647 862

## Description

### FIELD OF THE INVENTION

The present invention relates to apertured films suitable for use as topsheets or other fluid transporting layers in absorbent articles such as sanitary napkins and tampons. In particular, an apertured film topsheet receiving the liquids to be absorbed comprises at least two different, discrete, and visually distinct zones which do not overlap. These zones allow for the use of a single topsheet with different fluid passage characteristics and different tactile and visual characteristics within different zones of the topsheet.

### BACKGROUND OF THE INVENTION

Sanitary articles such as sanitary napkins, baby diapers, absorbent inserts, tampons, and absorbent adult incontinence articles are well-known in the art. Typically all these articles comprise a wearer facing surface and a garment facing surface. The wearer facing surface receives from the wearer of such articles bodily discharges such as urine, vaginal discharges or menses, to be absorbed. In order for the article to store the liquid, the wearer facing surface has to be liquid permeable while maintaining the integrity of the outer wearer facing surface of the absorbent article and preventing liquid absorbed by the article from flowing back out onto a wearer. This wearer facing surface is provided by a topsheet.

Well-known topsheets in the art of absorbent articles are non-woven fabrics and films. Non-woven fabrics are made of fibers which by their nature provide non-linear apertures for liquid transport, although such fabrics may further be modified with an arrangement of apertures. Films have to be rendered permeable by aperturing.

Films suitable for use as topsheets are generally made of polymeric material and typically comprise apertures or orifices which have been engineered to provide certain characteristics. These apertures are defined by sidewalls, which extend from the surface of the film. The apertures may vary in shape and size but have commonly been provided in a single preferred size and shape. An example of such a known topsheet is described in US 3,929,135 to Thompson. The sidewalls of the apertures define the amount of extension, if any, beyond the plane of the film thickness and the direction of such extensions. The sidewalls of the apertures also can be provided in the shape of a funnel.

Film topsheets are preferred over fabric topsheets by some users since they can provide a relatively cleaner-looking surface even after liquid has passed through since they do not generally retain liquids on their surfaces. However, some users may find film topsheets to be irritating or chafing, especially along the edges of the product, since the pattern (i.e., the size, shape, and spacing) of apertures provided by the topsheet extends across the entire topsheet, and the aperture pattern that is preferred for passing fluid quickly may not be preferred for facing against the user's inner thighs along the side edges of the product.

A method that has been used to overcome the disadvantages of side edge discomfort while retaining the benefits of quick fluid passage is that of using an apertured film topsheet over the entire surface of the absorbent product, and then covering the apertured film along the side edges of the product with a layer of nonwoven fabric. The apertured film provides good fluid handling properties in the center of the product where the fluid enters, and the nonwoven imparts greater comfort to the user by providing soft fabric along the product's edges. However, this adds material cost to the product and an extra step in the manufacturing process.

An additional disadvantage of known apertured films is that, because of their uniform aperture patterns, their fluid transport properties are generally uniform as well. Attempts have been made to improve the fluid transport characteristics of certain apertured films by the inclusion of larger apertures in and amongst the apertures of the uniform apertures pattern. However, this method still produces an apertured film with a uniform aperture arrangement, albeit an arrangement consisting of large holes and small holes. Furthermore, although the larger apertures may allow for the passage of fluid into an underlying absorbent layer, these same larger apertures may be more likely to allow the absorbed fluid to pass back out of the apertures when the wetted absorbent layer is subjected to pressure. In other words, absorbed fluid may be squeezed out of the absorbent layer, through the large holes, and onto the skin of a wearer of the absorbent article.

It is therefore an object of this invention to provide an apertured web suitable for use as an absorbent article topsheet with two or more different and discrete, that is, noncontinuous and individually distinct, zones within a singe topsheet.

The present invention provides an apertured film as defined in claim 1.

### BRIEF DESCRIPTION OF THE DRAWINGS

Fig. 1 is a top plan view of a sanitary napkin having a topsheet comprising an embodiment of the apertured web of the present invention.
Fig. 2 is an embodiment of an apertured web of the present invention.
Fig. 3 is a photomicrograph of a portion of the apertured web shown in Figure 2.
Fig. 4 is another embodiment of an apertured web of the present invention.
Fig. 5 is a photomicrograph of a portion of the apertured web shown in Figure 4.
Fig. 6 is yet another embodiment of an apertured web of the present invention.
Fig. 7 is a photomicrograph of a portion of the apertured web shown in Figure 6.
Fig. 8 is a photomicrograph of another portion of the apertured web shown in Figure 6.

### DESCRIPTION OF THE INVENTION

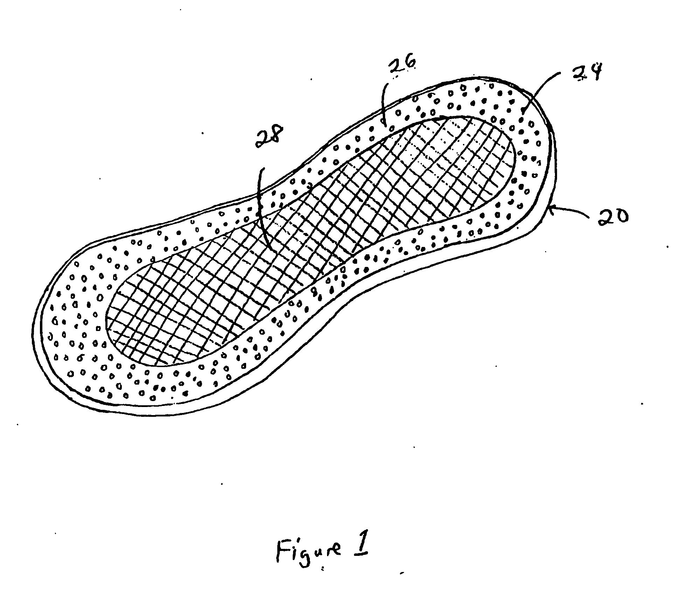

Referring now to the drawings, an absorbent article 20 having a topsheet 24 comprising an example of an apertured film of the present invention is shown in plan view in Figure 1.

It can be seen from Figure 1 that the apertured film of topsheet 24 has first zone 26 and second zone 28. First zone 26 is separate and distinct from second zone 28, that is, the first zone does not overlap the second zone. First zone 26 has a first arrangement of land areas and at least two apertures, and second zone 28 has a second arrangement of land areas and at least two apertures. The arrangement of land areas and at least two apertures of one zone does not continue into the other zone; i.e., the zones are discrete and separate.

The two zones are differentiated by their respective arrangements of apertures and land areas and the boundary between the first zone and the second zone may be defined as the place where the first arrangement ends and the second arrangement begins. Since the two arrangements are different, the boundary between them is not a natural border; that is, one arrangement does not naturally flow into the other arrangement. The boundary may be created by abruptly ending one arrangement and beginning another arrangement, or in some cases, by "morphing" the edges of the arrangements where they meet to form a boundary. Morphing means that the edges of the adjoining arrangements are modified slightly to allow for the arrangements to adjoin without abruptly ending one arrangement and beginning another arrangement, such as might occur if a cut edge of one arrangement were simply joined to a cut edge of another arrangement. Such morphed edges ease the transition from one arrangement to an adjoining arrangement. Morphed edges also help to prevent roughness in the boundary or weakness in the web in the boundary area. However, even when the edges are morphed, the boundary between the arrangements remains visually distinct. As used herein, "visually distinct" means visibly different to a normal, unaided, human eye at a distance of 12 inches between the eye and a visible object.

In the example shown in Figure 1, the first arrangement 26 ends and the second arrangement 28 begins along a boundary between the two arrangements. However, in alternate embodiments of the invention, the boundary between the two arrangements may have a third arrangement which is distinct and different from either the first or the second arrangements, or the boundary may be free of an arrangement of land areas and apertures.

Each zone is defined by an arrangement of land areas and at least two apertures, which provide the zone with an overall visual appearance. As used herein, "arrangement" means the specific size, distance, and orientation relationships among the land area and the at least two apertures in each zone. The arrangement may consist of one size or more than one size of aperture. Such an arrangement provides a zone with a distinct unified visual appearance, with no distinct break in its visual appearance, i.e., no separate boundary. Thus each arrangement be regular, irregular, random, repeating, nonrepeating, geometric, or nongeometric. As used herein, "regular" means recurring at fixed or regular intervals. "Irregular" as used herein, means not recurring at fixed or regular intervals. By "geometric" is meant an arrangement based on rectilinear or curvilinear motifs or outlines.

The apertured film of the present invention has at least two zones, each having an arrangement of land areas and at least two apertures. The zones are separated by a visibly distinct boundary.

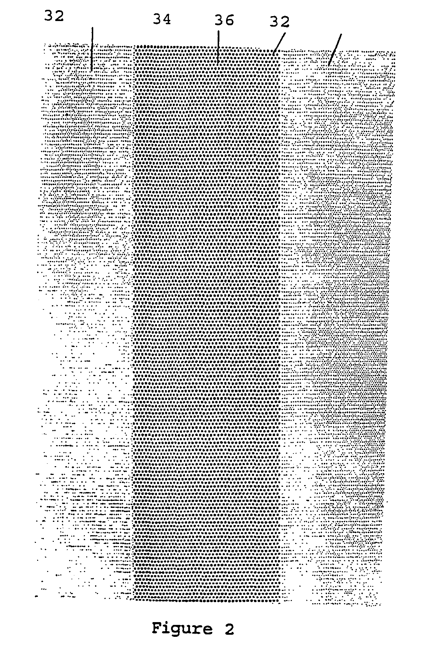

Referring now to Figure 2, an embodiment of an apertured web is shown. The first zone 32 has a first arrangement with a regular array of very small apertures, and the second zone 34 has a second arrangement with a regular array of somewhat larger apertures. When this apertured web is used as a topsheet on, for example, a sanitary napkin, the second zone 34 is positioned so that the incoming fluid tends to enter the article through the second zone. Thus, an arrangement of somewhat larger apertures is preferred for the second zone in this embodiment. In such a construction, the first zone 32 would be positioned along the side edges of the sanitary napkin, where smoothness against the inner thighs and groin area are a concern. The smaller aperture hole size and finer arrangement would provide a smoother surface in the first zone of this embodiment.

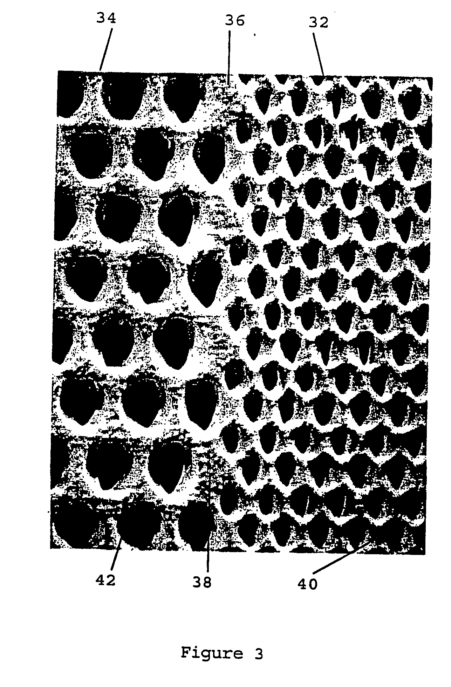

Figure 3 shows a photomicrograph of a portion of the apertured web shown in Figure 2. In particular, the portion of the web shown in Figure 3 shows a portion of first zone 32, a portion of second zone 34, and a portion of the boundary 36 that separates first zone 32 from second zone 34. It can be seen in Figure 3, that the boundary 36 is created by morphing the arrangement of the first zone 32 and the second zone 34. That is, the land areas 38 of the boundary are a different size and shape than the land areas 40 of the first zone 32 or the land areas 42 of the second zone 34.

Alternatively, another embodiment of an apertured film suitable for use as a topsheet on a sanitary napkin may have an arrangement of relatively smaller apertures in a central fluid-contacting region and relatively larger apertures along the sides of the napkin. In such a case, the aperture size in the central region may be chosen to allow the passage of fluid, and the aperture size along the sides may be chosen to minimize the land area of the arrangement and thus minimize contact with the skin of a wearer of such a napkin.

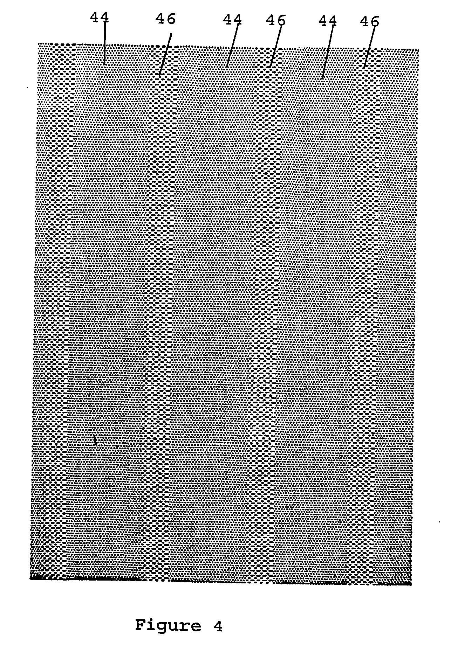

Figure 4 shows another embodiment of an apertured web. The web shown in Figure 4 has first zone 44 and second zone 46 in alternating arrangement. Such a web may be suitable for use as a topsheet on an absorbent article, such as a diaper or a sanitary napkin, where the alternating zones tend to distribute incoming fluid over the topsheet for passage through the topsheet and into an underlying absorbent material. Such distribution helps to utilize more of the absorbent material than if incoming fluid passed directly through the apertured web at its point of impact. Utilization of more of the absorbent material helps to prevent leakage of fluid out of the product and to prevent puddling, or wetness of the topsheet caused by oversaturation of the underlying absorbent material and passage of previously absorbed fluid back through to the surface of the topsheet.

The apertured web shown in Figure 4 is also suitable for use as a topsheet on a catamenial tampon. The web may be slit so that second zone 46 overlies the center of the tampon's outer surface, and first zone 44 overlies the outer surface of the leading end and the trailing end of the tampon.

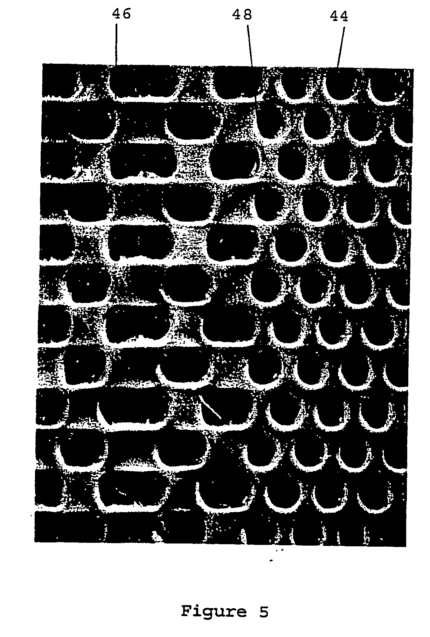

Figure 5 is a photomicrograph of a portion of the apertured web of Figure 4. First zone 44 and second zone 46 are separated from one another by boundary 48. It can be seen that first zone 44 and second zone 46 are discrete and separate from one another with neither arrangement of land areas and apertures continuing into the other arrangement.

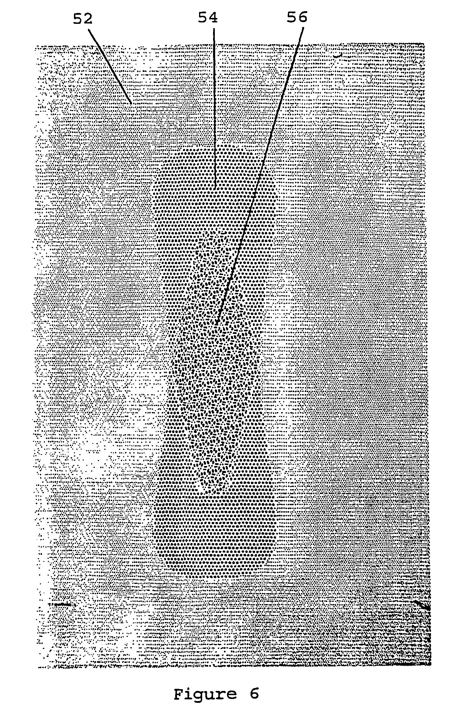

Referring now to Figure 6, an embodiment of an apertured web is shown with first zone 52, second zone 54, and third zone 56. The embodiment shown in Figure 6 is suitable for use as a topsheet or other fluid passage layer in an absorbent article such as a sanitary napkin or a diaper. In such a use, third zone 56 is positioned in the region of the article where fluid tends to enter the article, and thus, the arrangement of land areas and apertures of third zone 56 is chosen to pass fluid quickly and provide a dry surface to a wearer of the article. The arrangement of second zone 54 is chosen to provide a comfortable surface to a wearer and to pass any fluid that is not passed by third zone 56. The arrangement of first zone 56 comprises small apertures, which present a smooth silky surface to the skin of a wearer.

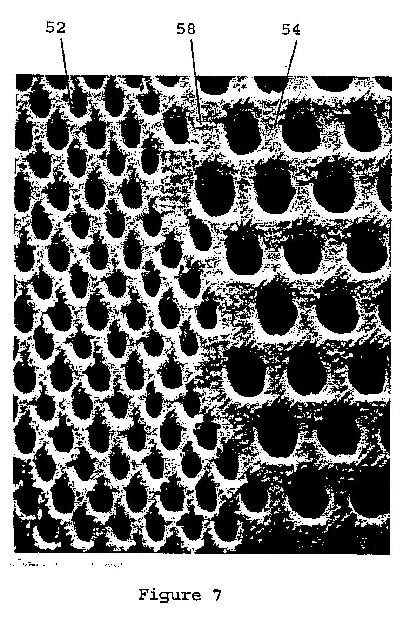

Figure 7 is a photomicrograph of a portion of the apertured web of Figure 6, showing first zone 52 and second zone 54 separated from one another by boundary 58.

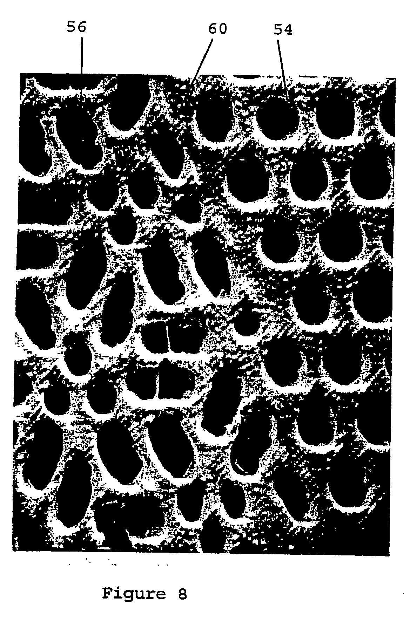

Figure 8 is a photomicrograph of another portion of the apertured web of Figure 6, showing second zone 54 and third zone 56 separated from one another by boundary 60.

In another embodiment, a first zone may have an arrangement of land areas and at least two apertures so spaced that said arrangement is larger in dimension than the space defined by the boundary separating the first zone from a second zone. In other words, the arrangement is not repeated within the zone.

A suitable film of the present invention is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed or before the film is formed by incorporating the surface active agent as a blend into the thermoplastic polymeric material. The film may comprise any thermoplastic polymeric material including, but not limited to, polyolefins, such as high density polyethylene, linear low density polyethylene, low density polyethylene, polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate and ethylenemethacrylate. Films comprising mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface active agent. The starting film can be made by any known technique, such as casting, extrusion, or blowing.

An apertured film of this invention may be formed by placing a thermoplastic film across the surface of an apertured support member with an arrangement of apertures. A stream of hot air is directed against the film to raise its temperature to cause it to be softened. A vacuum is then applied to the film to cause it to conform to the shape of the surface of the support member. Portions of the film lying over the apertures in the support member are ruptured to create apertures in the film.

An apertured support member suitable for making the multiple zone apertured webs of this invention is made by raster scan drilling, as described in U.S. 5,916,462 to James et al.

## Claims

1. An apertured film comprising:
a first zone (26, 32, 44, 52) comprising a first arrangement of land areas (40) and at least two apertures, the apertures having the same size as each other, and
a second zone (28, 34, 46, 54) comprising a second arrangement of land areas (42) and at least two apertures, the apertures having the same size as each other, but different in size than the apertures of the first zone, and
wherein the first zone (26, 32, 44, 52) is discrete and separate from, and does not overlap, the second zone (28, 34, 46, 54), wherein said first and second apertures have sidewalls extending from a surface of the film, and wherein the apertured film is a body-facing topsheet (24) suitable for an absorbent article (20).

2. The film of claim 1 wherein the first arrangement is regular.

3. The film of claim 1 wherein the first arrangement is irregular.

4. The film of claim 1 wherein the first arrangement is repeating.

5. The film of claim 1 wherein the first arrangement is nonrepeating.

6. The film of claim 1 wherein the first arrangement is geometric.

7. The film of claim 1 wherein the first arrangement is morphed along a boundary (36, 48, 60) between the first zone (26, 32, 44, 52) and the second zone (28, 34, 46, 54).

8. The film of any one of claims 1 to 7 further comprising at least one third zone (56) comprising a third arrangement of land areas and at least two apertures.

9. An absorbent article (20) comprising the apertured film of any one of claims 1 to 8.

10. The absorbent article (20) of claim 9 which is a sanitary napkin, a diaper or a tampon.

11. The absorbent article (20) of claim 9 or claim 10, wherein the first zone (26, 32) is positioned in a central region for receiving fluid into said absorbent article (20), and wherein said second zone (28, 34) is positioned along a side edge of said absorbent article (20).

## Patentansprüche

1. Perforierte Folie, Folgendes umfassend:
eine erste Zone (26, 32, 44, 52), die eine erste Anordnung von Stegbereichen (40) und mindestens zwei Perforationen umfasst, wobei die Perforationen gleich groß sind, und
eine zweite Zone (28, 34, 46, 54), die eine zweite Anordnung von Stegbereichen (42) und mindestens zwei Perforationen umfasst, wobei die Perforationen gleich groß sind, aber eine andere Größe aufweisen als die Perforationen der ersten Zone, und
wobei die erste Zone (26, 32, 44, 52) von der zweiten Zone (28, 34, 46, 54) verschieden und getrennt ist und diese nicht überlappt, wobei die ersten und die zweiten Perforationen Seitenwandungen aufweisen, die sich von einer Oberfläche der Folie aus erstrecken, und wobei die perforierte Folie eine dem Körper zugewandte Decklage (24) ist, die für einen absorbierenden Gegenstand (20) geeignet ist.

2. Folie nach Anspruch 1, wobei die erste Anordnung regelmäßig ist.

3. Folie nach Anspruch 1, wobei die erste Anordnung unregelmäßig ist.

4. Folie nach Anspruch 1, wobei sich die erste Anordnung wiederholt.

5. Folie nach Anspruch 1, wobei sich die erste Anordnung nicht wiederholt.

6. Folie nach Anspruch 1, wobei die erste Anordnung geometrisch ist.

7. Folie nach Anspruch 1, wobei die erste Anordnung entlang einer Begrenzung (36, 48, 60) zwischen der ersten Zone (26, 32, 44, 52) und der zweiten Zone (28, 34, 46, 54) in der Gestaltung verändert ist.

8. Folie nach einem der Ansprüche 1 bis 7, ferner mindestens eine dritte Zone (56) umfassend, die eine dritte Anordnung von Stegbereichen und mindestens zwei Perforationen umfasst.

9. Absorbierender Gegenstand (20), die perforierte Folie nach einem der Ansprüche 1 bis 8 umfassend.

10. Absorbierender Gegenstand (20) nach Anspruch 9, der eine Damenbinde, eine Windel oder ein Tampon ist.

11. Absorbierender Gegenstand (20) nach Anspruch 9 oder Anspruch 10, wobei die erste Zone (26, 32) in einem mittleren Bereich angeordnet ist, um Flüssigkeit im absorbierenden Gegenstand (20) aufzunehmen, und wobei die zweite Zone (28, 34) entlang eines Seitenrandes des absorbierenden Gegenstandes (20) angeordnet ist.

## Revendications

1. Film perforé, comprenant:
une première zone (26, 32, 44, 52) comprenant un premier agencement de surfaces d'appui (40) et au moins deux ouvertures, les ouvertures ayant la même taille; et
une deuxième zone (28, 34, 46, 54) comprenant un deuxième agencement de surfaces d'appui (42) et au moins deux ouvertures, les ouvertures ayant la même taille mais une taille différente de celle des ouvertures de la première zone; et
dans lequel la première zone (26, 32, 44, 52) est discrète et séparée de, et ne chevauche pas, la deuxième zone (28, 34, 46, 54), dans lequel lesdites première et deuxième ouvertures présentent des parois latérales qui s'étendent à partir d'une surface du film, et dans lequel le film perforé est une feuille supérieure côté corps (24) appropriée pour un article absorbant (20).

2. Film selon la revendication 1, dans lequel le premier agencement est régulier.

3. Film selon la revendication 1, dans lequel le premier agencement est irrégulier.

4. Film selon la revendication 1, dans lequel le premier agencement est répétitif.

5. Film selon la revendication 1, dans lequel le premier agencement est non répétitif.

6. Film selon la revendication 1, dans lequel le premier agencement est géométrique.

7. Film selon la revendication 1, dans lequel le premier agencement est formé le long d'une frontière (36, 48, 60) entre la première zone (26, 32, 44, 52) et la deuxième zone (28, 34, 46, 54).

8. Film selon l'une quelconque des revendications 1 à 7, comprenant en outre au moins une troisième zone (56) comprenant un troisième agencement de surfaces d'appui et au moins deux ouvertures.

9. Article absorbant (20) comprenant le film perforé selon l'une quelconque des revendications 1 à 8.

10. Article absorbant (20) selon la revendication 9 qui est une serviette hygiénique, une couche ou un tampon.

11. Article absorbant (20) selon la revendication 9 ou la revendication 10, dans lequel la première zone (26, 32) est positionnée dans une région centrale destinée à recevoir un fluide dans ledit article absorbant (20), et dans lequel ladite deuxième zone (28, 34) est positionnée le long d'un bord latéral dudit article absorbant (20).
